# EUROPÄISCHE PATENTSCHRIFT

(11) **EP 2 103 287 B1**
(45) Veröffentlichungstag und Bekanntmachung des Hinweises auf die Patenterteilung: **07.09.2011**
(21) Anmeldenummer: 09003461.2
(22) Anmeldetag: 10.03.2009
(51) Int. Cl.: A61F 5/30, A61F 13/06, A61F 13/10

(54) **Pelotte**
Pelotte
Pelote

(30) Priorität: 22.03.2008 DE 102008015449
(43) Veröffentlichungstag der Anmeldung: 23.09.2009
(73) Patentinhaber: Lohmann & Rauscher GmbH & Co. KG, 56567 Neuwied (DE)
(72) Erfinder: Sandhof, Marc Christian, 56579 Rengsdorf (DE)
(74) Vertreter: Flaccus, Rolf-Dieter

(56) Entgegenhaltungen:
- WO-A-01/56516
- WO-A-03/020186
- US-A- 2 896 612

## Beschreibung

Die Erfindung betrifft eine Pelotte mit einer speziellen Oberflächenstruktur.

Pelotten werden in der Regel zu medizinischen Zwecken, insbesondere zur Massage und Stimulation von Körperbereichen oder zur lymphologischen Behandlung oder Anregung genutzt. Sie bestehen im Allgemeinen aus einem elastischen Material und sind meist in Bandagen eingearbeitet, die über den zu behandelnden Körperteil gestreift werden. Solche Pelotten enthaltenden Bandagen sind dem Fachmann z.B. aus DE 10 2005 062 477, DE 20 2004 013 159 U, US 5,135,473 oder FR 2 607 383 A1 bekannt.

WO 03/020186 offenbart eine Vorrichtung zur Behandlung einer erhöhten Konzentration von interstitieller Flüssigkeit im Körper eines Patienten. Die Vorrichtung ist eine elastische Auflage bei der sich im Inneren einer Umhüllung elastische Vorsprünge befinden.

US 2,896,612 befasst sich mit einer Pelotte bestehend aus wellenförmig angeordneten Schläuchen in denen wechselseitig ein Druck aufgebaut wird, was einen aktiven Pumpvorgang bei Körperflüssigkeiten stimuliert.

Um eine optimale Druckverteilung und damit eine gezielte Massagewirkung zu erreichen, bestehen die Pelotten meist aus Silikonpolstem und sind manchmal an ihrer Oberfläche genoppt. Solche genoppten Petotten sind z.B. aus DE 202 03 275 U 1 bekannt.

Pelotten mit einer glatten oder genoppten Oberfläche sind jedoch oftmals ineffizient, was den Abbau von Schwellungen durch ihre Massagewirkung, insbesondere ihre Fähigkeiten zur lymphologischen Behandlung oder Anregung, betrifft. Dies liegt darin begründet, dass diese glatten oder genoppten Oberflächen in ihrer Struktur homogen sind und somit einen gezielten Abfluss der Lymphflüssigkeit in einer bevorzugten Richtung längs der Lymphkanäle nicht optimal unterstützen.

Aufgabe der vorliegenden Erfindung ist es, die oben genannten Nachteile zu überwinden und eine Pelotte bereitzustellen, die eine verbesserte Massagewirkung, insbesondere hinsichtlich der lymphologischen Behandlung oder Anregung, aufweist.

Diese Aufgabe wird durch eine Pelotte nach Anspruch 1 gelöst. Diese Pelotte besteht aus einem Kissen aus einem elastischen Material und weist zumindest auf Teilen ihrer Oberfläche eine periodische Oberflächenstruktur aus langgezogenen Elementen auf.

Vorzugsweise ist diese Oberflächenstruktur wellenartig, daher werden die langgezogenen Elemente im Folgenden als "Wellenzüge" bezeichnet, obwohl sie z.B. auch die Form von Rechteck- oder Zickzackmustern annehmen können. Die periodische Oberflächenstruktur wird im Folgenden der besseren Anschauung halber auch als wellenartige Oberflächenstruktur bezeichnet, was jedoch andere periodische Verläufe, wie z.B. Rechteck- oder Zickzackmuster, nicht ausschließt.

Die Oberflächenstruktur besteht aus erhöhten Wellenzügen, die jeweils durch Zwischenräume getrennt sind. Diese Wellenzüge stellen Strukturen dar, die in einer X/Y-Ebene verlaufen, wobei sowohl die X-Richtung als auch die Y-Richtung parallel zur Oberfläche der Pelotte stehen. Die Y-Richtung liegt dabei orthogonal zur X-Richtung. Bevorzugte Formen der Wellenzüge sind die Form von Sinusfunktionen oder von Überlagerungen von beliebig vielen Sinusund Cosinusfunktionen beliebiger Phase und Wellenlänge in einer Fourierreihe, vorzugsweise mit jeweils ganzzahligen Vielfachen der Phase und Wellenlänge einer Basisfunktion. Der Ausdruck "beliebig vielen" bedeutet dabei hier und im Folgenden in diesem Zusammenhang, dass so viele Funktionen überlagert werden, wie der Fachmann für richtig erachtet, vorzugsweise bedeutet dies 1 bis 10 Sinus- und Cosinusfunktionen.

Bei einer Betrachtung der Oberfläche der Pelotte von oben ergeben die verschiedenen Wellenzüge somit ein Muster aus nebeneinander angeordneten Wellen oder anderen periodischen Strukturen.

Die Wellenlänge eines Wellenzuges beträgt zwischen 1 mm und 30 cm, vorzugsweise zwischen 0,5 cm und 3 cm. Die Amplitude des Wellenzuges beträgt zwischen 0,1 mm und 3 cm, vorzugsweise zwischen 1 mm und 1 cm.

Die einzelnen Wellenzüge sind durch Zwischenräume voneinander getrennt. Eine Anzahl aus mehreren Wellenzügen und Zwischenräumen bildet die wellenartige Oberflächenstruktur der Pelotte. Der Abstand der Wellenzüge beträgt zwischen 0,1 mm und 3 cm, vorzugsweise zwischen 1 mm und 1 cm. Im Unterschied zu den Zwischenräumen ragen die Wellenzüge zwischen 0,1 mm und 3 cm, vorzugsweise zwischen 1 mm und 1 cm, über der Oberfläche empor.

Der Querschnitt durch einen solchen wellenartigen Oberflächenbereich mit Wellenzügen und Zwischenräumen ähnelt wieder einer periodischen Struktur, vorzugsweise einer weiteren Wellenstruktur. Die Querschnittsform der erhöhten Wellenzüge und der Zwischenräume entspricht vorzugsweise versetzt angeordneten Halbkreisen, Rechtecken, Zickzackmustern, Sinuswellen oder Vielecken mit oder ohne abgerundeten Ecken. Im Allgemeinen sind jedoch Überlagerungen von beliebig vielen Sinus- und Cosinusfunktionen beliebiger Phase und Wellenlänge in einer Fourierreihe, vorzugsweise mit jeweils ganzzahligen Vielfachen der Phase und Wellenlänge einer Basisfunktion, als mögliche Formen denkbar. Die Höhe der Wellenzüge beträgt zwischen 0,1 mm und 3 cm, vorzugsweise zwischen 1 mm und 1 cm.

Zum besseren Verständnis sei hier angemerkt, dass die einzelnen Erhöhungen auf der Oberfläche der Pelotte im Folgenden sowohl als "Wellenzüge", als auch als "Wellenfronten" bezeichnet werden. Der Terminus "Wellenfront" wird dabei benutzt, um die Ausrichtung der Wellenzüge auf der Oberfläche zu beschreiben und entspricht der oben genannten "Richtung X". Die Zwischenräume werden im Folgenden auch als "Wellentäler" bezeichnet.

In einer bevorzugten Ausführungsform weist das Höhenprofil mindestens einer Wellenfront ebenfalls einen periodischen oder unregelmäßigen Verlauf auf. Längs der Länge eines Wellenzuges hat dieser in einer Richtung orthogonal zu der oben erwähnten X/Y-Ebene (Oberfläche der Pelotte) die Form einer periodischen Struktur, vorzugsweise die Form von Überlagerungen von beliebig vielen Sinus- und Cosinusfunktionen beliebiger Phase und Wellenlänge in einer Fourierreihe, besonders bevorzugt mit jeweils ganzzahligen Vielfachen der Phase und Wellenlänge einer Basisfunktion. Insbesondere bestehen diese Formen aus einer Sinuswelle, versetzt angeordneten Halbkreisen, Rechtecken, Zickzackmustern, oder Vielecken mit oder ohne abgerundeten Ecken. Der Höhenunterschied längs eines Wellenzuges beträgt zwischen 0,01 mm und 3 cm, vorzugsweise zwischen 1 mm und 1 cm.

Dieses periodische Höhenprofil der Wellenzüge spiegelt sich in einer bevorzugten Ausführungsform auch in den Zwischenräumen wieder, so dass auf der Pelotte das Bild einer oben genannten normalen, periodischen Oberflächenstruktur entsteht, die mit einer welligen Pelottenoberfläche interferiert.

Die wellenartigen Oberflächenstrukturen sind in die Oberfläche eingearbeitet oder auf der Oberfläche aufgebracht. In bevorzugten Ausführungsformen sind die wellenartigen Oberflächenstrukturen bereits in der Form zur Herstellung der Pelotte enthalten oder werden nach der Herstellung der Pelotte in Form von einzelnen Wellenzügen oder als eine mit Wellenzügen strukturierte Folie oder als strukturiertes Textil auf die Pelotte aufgeklebt oder mit dieser verschweißt.

Diese Oberflächenstruktur ist vorzugsweise so auf der Oberfläche angeordnet, dass die Wellenberge und Wellentäler in Abhängigkeit zu den überdeckten Lymphbahnen parallel zur Oberfläche der Pelotte in Form von liegenden Wellen relativ zur Oberfläche ausgerichtet sind.

Eine solche wellige Struktur bildet bei der Anwendung der Pelotte auf der Haut kleine Kanäle aus, zwischen denen die Wellenzüge liegen, welche auf die Hautschichten drücken. Hierbei wird durch Kompression oder Massage Lymphflüssigkeit entlang der Kanäle (Wellentäler) geleitet, weil in deren Bereich die Kompressionswirkung der Pelotte geringer ist als in den Bereichen der Wellenzüge. Entspricht diese Richtung in etwa dem natürlichen Verlauf der Lymphgefäße, so findet ein optimaler Lymphabfluss statt.

Die Wellenfronten sind dabei vorzugsweise in einem Winkelbereich von +45° bis -45°, vorzugsweise +10° bis -10°, relativ zu dem
anatomischen Verlauf der Lymphgefäße der überdeckten Körperstelle gemäß der Fachliteratur oder bezüglich des Patienten, angeordnet. Mit dem Verlauf der Lymphgefäße ist dabei sowohl der Verlauf der Hauptkanäle des Lymphsystems als auch die Vorzugsrichtung aller Lymphgefäße der überdeckten Stelle gemeint.

Der Verlauf der Lymphgefäße in einem menschlichen Körper ist einem Fachmann bekannt. Die individuelle Anordnung der Lymphgefäße im Körper eines Patienten ist dabei nicht unbedingt entscheidend, da durch die wellige Form der Oberfläche der Pelotte Abweichungen vom theoretischen Verlauf kompensiert werden. Die Wellenstruktur hat gegenüber geradlinigen Strukturen noch den besonderen Vorteil, dass bei Bewegung und dem damit verbundenen Verschieben der Pelotte auf der Haut eine Pumpwirkung längs der Wellentäler erfolgt.

In einer bevorzugten Ausführungsform sind die einzelnen Wellenzüge auf einer Pelotte längs der Richtung der Wellenfronten nicht unterbrochen, um einen optimalen Abfluss der Lymphflüssigkeit längs der Kanäle ohne Unterbrechungen zu gewährleisten.

Materialien, die zur Herstellung einer Pelotte geeignet sind, sind dem Fachmann bekannt. Bevorzugte Materialien sind aus einer Gruppe ausgewählt, die weichelastisch nicht kompressible Materialien, weichelastisch kompressible Materialien oder Kombinationen der zuvor genannten Materialien umfasst. Solche Materialien können Kunststoffe oder natürliche Stoffe sein, und auch Gewirke, Gewebe oder Abstandsgewebe, Gelege, Schaumstoffe, Vliese und vergleichbare Materialien enthalten. Beispiele für weichelastische nicht kompressible Materialien sind z.B. die Silikone und Kautschuke, während Kunststoffschäume wie Silikon- und Polymerschäume zu den weichelastisch kompressiblen Materialien gehören.

Weitere bevorzugte Materialien sind Syntheselatex oder Naturlatex, Neopren, Polyethylen (HDPE oder LDPE), Polypropylen oder ein Copolymer Polypropylen. Die Elastizität aller aufgeführten Materialien kann dabei durch die Einarbeitung von beispielsweise Elastan oder Elastodien verändert werden.

In einer bevorzugten Ausführungsform wird die Pelotte in Bandagen angeordnet. Solche Bandagen werden insbesondere zur Behandlung der Achillessehne eingesetzt, bevorzugt ist aber auch der Einsatz zur Behandlung weiterer Extremitäten, die lymphologisch behandelt oder angeregt werden sollen, wie zum Beispiel an der Schulter, am Arm, an der Hand, am Rumpf, an der Hüfte, am Knie, am Unterschenkel, am Knöchel oder am Fuß.

In einer weiteren bevorzugten Ausführungsform weist die Pelotte eine zusätzliche Lochung auf, die eine Schweißbildung unterdrückt und eine zusätzliche Massagewirkung entfaltet. Insbesondere bei einer Pelotte für eine Achillessehnen-Bandage verläuft eine solche Lochung bevorzugt entlang der Achillessehne und verhindert so die Schweißbildung im Sprunggelenkbereich.

In FIG. 1 ist die wellenartige Struktur auf einer Pelotte in einer Aufsicht dargestellt.

FIG. 2 zeigt vier verschiedene mögliche Querschnitte der wellenartigen Struktur.

In FIG. 3 ist ein Beispiel einer Pelotte für eine Achillessehnen-Bandage mit der wellenartigen Oberflächenstruktur skizziert.

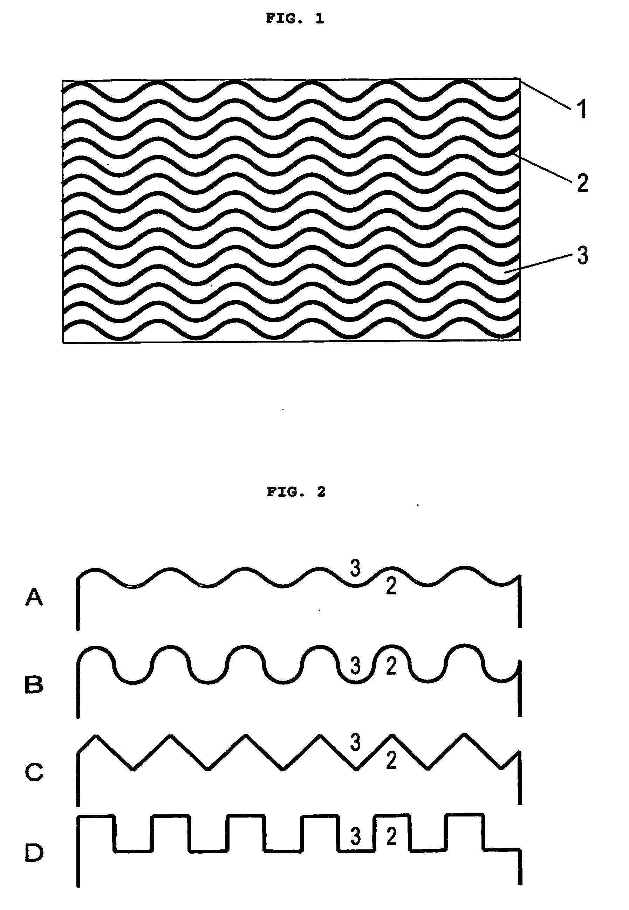

Ein Beispiel einer oben beschriebenen wellenartigen Oberflächenstruktur ist in FIG. 1 dargestellt. In der Aufsicht auf die wellige Oberfläche einer Pelotte (1) sind schwarz die Wellenzüge (2) zu erkennen, die durch weiße Zwischenräume (3) getrennt sind.

Vier mögliche Querschnittsformen der Wellenzüge (2) sind in FIG. 2 dargestellt. In Beispiel A haben dabei die Wellenzüge (2) und Zwischenräume (3) die Form einer Sinusfunktion, in Beispiel B die Form von Halbkreisen, in Beispiel C eine Zickzackform und in Beispiel D eine Rechteckform. Aufgrund der Elastizität des Materials der Pelotte sind Formen mit Kanten durchaus möglich, ohne dass eine Einschränkung der Bequemlichkeit zu erwarten wäre.

Ein Beispiel einer Pelotte für eine Achillessehnen-Bandage ist im Folgenden dargestellt:

Die Pelotte sollte von ihrer Form her so gestaltet sein, dass sie Unebenheiten, die durch die anatomischen Strukturen am Unterschenkel oder am Fuß vorhanden sind, ausgleicht. Eine solche Grundform ist dem Fachmann bekannt.

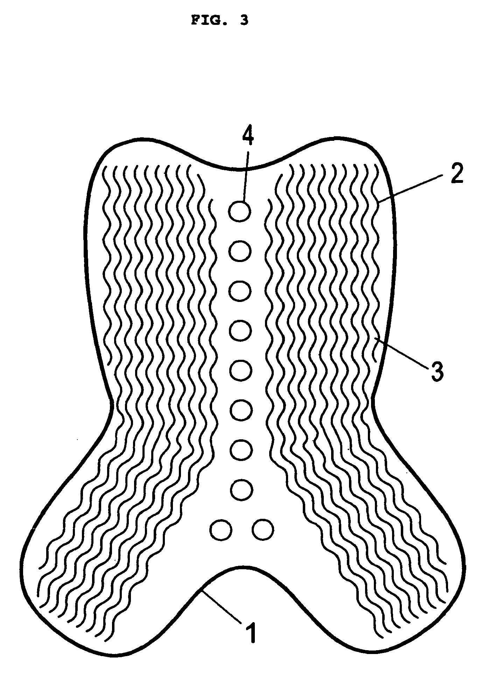

Dadurch wird eine gleichmäßigere Kompression durch die Bandage auf dem Körper erreicht. Diese Form legt sich medial und lateral auf die Ferse und verläuft nach dorsal über die Achillessehne, wobei die Pelotte ca. 4 cm zu beiden Seiten die Sehne einfasst. Eine solche Pelotte (1) ist in FIG. 3 dargestellt.

Die Oberfläche der Pelotte weist in zwei Teilbereichen eine wellenartige Oberflächenstruktur auf, bestehend aus mehreren Wellenzügen (2), die durch Zwischenräume (3) getrennt sind. Die Wellenzüge sind dabei in der Richtung des Verlaufs der Bahnen des dorso-lateralen Lymphbündels angeordnet. Zusätzlich sind längs der Mitte Löcher (4) in der Pelotte angeordnet, um eine Schweißbildung zu unterdrücken und eine zusätzliche Massagewirkung zu entfalten.

Bei einer Anwendung einer solchen Pelotte in einer Achillessehnen-Bandage wird durch Kompression und Massage Lymphflüssigkeit aus dem Interstitium über die Bahnen des dorso-lateralen Lymphbündels und somit von der Peripherie über die Vena saphena parva dem Venensystem zugeführt und letztendlich ausgeschieden.

## Patentansprüche

1. Pelotte (1), bestehend aus einem Kissen aus einem elastischen Material, wobei Teile ihrer Oberfläche oder die gesamte Oberfläche der Pelotte (1) eine periodische Oberflächenstruktur aus erhöhten Wellenzügen (2) aufweisen, die durch Zwischenräume (3) getrennt sind und in einer X/Y-Ebene verlaufen, wobei sowohl die X-Richtung als auch die Y-Richtung parallel zur Oberfläche der Pelotte (1) stehen, wobei der Querschnitt durch die periodische Oberflächenstruktur der Form von versetzt angeordneten Halbkreisen, Rechtecken, Zickzackmustern, Sinuswellen oder Vielecken mit oder ohne abgerundeten Ecken entspricht, **dadurch gekennzeichnet dass** die Pelotte (1) eine zusätzliche Lochung aufweist.

2. Pelotte (1) nach Anspruch 1, **dadurch gekennzeichnet, dass** die Wellenzüge (2) die Form von Sinusfunktionen oder von Überlagerungen von beliebig vielen Sinus- und Cosinusfunktionen beliebiger Phase und Wellenlänge in einer Fourierreihe, vorzugsweise mit jeweils ganzzahligen Vielfachen der Phase und Wellenlänge einer Basisfunktion haben, wobei die Wellenlänge eines Wollenzugen (2) vorzugsweise zwischen 1 mm und 30 cm, besonders bevorzugt zwischen 0,5 cm und 3 cm, und die Amplitude des Wellenzuges (2) vorzugsweise zwischen 0,1 mm und 3 cm beträgt, besonders bevorzugt zwischen 1 mm und 1 cm.

3. Pelotte (1) nach einem der vorangehenden Ansprüche, **dadurch gekennzeichnet, dass** der Abstand der Wellenzüge (2) zwischen 0,1 mm und 3 cm, vorzugsweise zwischen 1 mm und 1 cm, beträgt und die Wellenzüge (2) im Unterschied zu den Zwischenräumen (3) zwischen 0,1 mm und 3 cm, besonders bevorzugt zwischen 1 mm und 1 cm, aus der Oberfläche emporragen.

4. Pelotte (1) nach einem der vorangehenden Ansprüche, **dadurch gekennzeichnet, dass** die periodische Oberflächenstruktur in die Oberfläche eingearbeitet oder auf die Oberfläche aufgebracht ist, wobei die periodische Oberflächenstruktur bevorzugt bereits in der Form zur Herstellung der Pelotte (1) enthalten ist oder nach der Herstellung der Pelotte (1) in form von einzelnen Wellenzügen (2) oder als eine mit Wellenzügen (2) strukturierte Folie oder als mit Wellenzügen (2) strukturiertes Textil auf die Pelotte (1) aufgeklebt ist oder mit dieser verschsweißt ist.

5. Pelotte (1) nach einem der vorangehenden Ansprüche, **dadurch gekennzeichnet, dass** die periodische Oberflächenstruktur Materialien enthält, die aus der Gruppe ausgewählt sind, die weichelastisch nicht kompressible Materialien, weichelastisch kompressible Materialien und nicht verformbare Materialien oder Kombinationen der zuvor genannten Materialien umfasst, bevorzugt Kunststoffe oder natürliche Stoffe, Gewirke, Gewebe oder Abstandsgewebe, Gelege, Schaumstoffe, Vliese oder vergleichbare Materialien, besonders bevorzugt Silikone und Kautschuke, Silikon- und Polymerschäume, Syntheselatex, Naturlatex, Neopren, Polyethylen, Polypropylen oder ein Copolymer PP.

6. Pelotte (1) nach einem der vorangehenden Ansprüche, **dadurch gekennzeichnet, dass** die Pelotte (1) in einer Bandage angeordnet ist, bevorzugt in Bandagen zur Behandlung der Achillessehne, der Schulter, des Arms, der Hand, des Rumpfes, der Hüfte, des Knies, des Unterschenkels, des Knöchels oder des Fußes.

7. Pelotte (1) nach einem der vorangehenden Ansprüche, **dadurch gekennzeichnet, dass** die periodische Oberflächenstruktur aus erhöhten Wellenzügen (2) besteht, die jeweils durch Zwischenräume (3) getrennt sind, wobei jeder dieser wollenzüge (2) eine liegende Welle parallel zur Oberfläche der Pelotte (1) bildet.

8. Pelotte (1) nach einem der vorangehenden Ansprüche, **dadurch gekennzeichnet, dass** das Höhenprofil mindestens einer Wellenfront einen periodischen oder unregelmäßigen Verlauf orthogonal zur Oberfläche der Pelotte (1), entlang ihrer Länge aufweist, vorzugsweise die Form von Überlagerungen von beliebig vielen Sinus- und Cosinusfunktionen beliebiger Phase und Wellenlänge in einer Fourierreihe hat, besonders bevorzugt mit jeweils ganzzahligen Vielfachen der Phase und Wellenlänge einer Basisfunktion, insbesondere die Form einer Sinuswelle, versetzt angeordneten Halbkreisen, Rechtecken, Zickzackmustern, oder Vielecken mit oder ohne abgerundeten Ecken hat, wobei bevorzugt der Höhenunterschied längs eines Wellenzuges (2) zwischen 0,01 mm und 3 cm, vorzugsweise zwischen 1 mm und 1 cm beträgt.

9. Pelotte (1) nach einem der vorangehenden Ansprüche, **dadurch gekennzeichnet, dass** die einzelnen Wellenzüge (2) auf der Pelotte (1) längs ihres Verlaufes nicht unterbrochen sind.

10. Verfahren der Verstellung einer Pelotte (1) aus einem Kissen aus einem elastischen Material, **dadurch gekennzeichnet, dass** Teile ihrer Oberfläche oder die gesamte Oberfläche der Pelotte (1) eine periodische Oberflächenstruktur nach einem der vorangehenden Ansprüche aufweist und dass die wellenartigen Oberflächenstrukturen in die Oberfläche eingearbeitet werden oder auf die Oberfläche aufgebracht werden, wobei die periodischen Oberflächenstrukturen bevorzugt bereits in der Form zur Herstellung der Pelotte (1) enthalten sind oder nach der Herstellung der Pelotte (1) in Form von einzelnen Wellenzügen (2) oder als eine mit Wellenzügen (2) strukturierte Folie oder als mit Wellenzügen (2) strukturiertes Textil auf die Pelotte (1) aufgeklebt werden oder mit dieser verschweißt werden.

## Claims

1. Pelotte (1), consisting of a cushion made of an elastic material, wherein portions of the surface or the entire surface of the pelotte (1) exhibit(s) a periodic surface structure of elevated wave trains (2), which wave trains (2) are separated by gaps (3) and run in an X/Y plane, both the X direction and the Y direction being parallel to the surface of the pelotte (1), and wherein the cross section through the periodic surface structure corresponds to the shape of semi-circles, rectangles, zigzag patterns, sine waves or polygons, with or without rounded corners, in offset arrangement, **characterised in that** the pelotte (1) in addition exhibits a perforation.

2. Pelotte (1) according to claim 1, **characterised in that** the wave trains (2) have the shape of sine functions or superpositions of any number of sine and cosine functions of any phase and wavelength in a Fourier series, preferably, each with integral multiples of the phase and wavelength of a basis function, wherein the wavelength of a wave train (2) is preferably from 1 mm to 30 cm, more preferably from 0.5 cm to 3 cm, and the amplitude of the wave train (2) is preferably from 0.1 mm to 3 cm, more preferably from 1 mm to 1 cm.

3. Pelotte (1) according to any one of the preceding claims, **characterised in that** the distance between the wave trains (2) is from 0.1 mm to 3 cm, preferably from 1 mm to 1 cm, and the wave trains (2), by contrast to the gaps (3), protrude from the surface by 0.1 mm to 3 cm, especially preferably by 1 mm to 1 cm.

4. Pelotte (1) according to any one of the preceding claims, **characterised in that** the periodic surface structure is integrated in the surface or is applied thereto, with the periodic surface structure preferably already being contained in the mould employed for the manufacture of the pelotte (1), or, after the manufacture of said pelotte (1), being bonded, or welded, to the pelotte (1) in the form of individual wave trains (2) or as a sheet structured with wave trains (2) or as a textile fabric structured with wave trains (2).

5. Pelotte (1) according to any one of the preceding claims, **characterised in that** the periodic surface structure contains materials selected from the group comprising soft elastically incompressible materials, soft elastically compressible materials and non-ductile materials, or combinations of the aforementioned materials, preferably synthetic materials or natural materials, knit fabrics, woven fabrics or spacer fabrics, interlaid scrims, foamed plastics, nonwovens or comparable materials, more preferably silicones and rubbers, silicone foams and polymer foams, synthetic latex, natural latex, neoprene, polyethylene, polypropylene or a copolymer PP.

6. Pelotte (1) according to any one of the preceding claims, **characterised in that** the pelotte (1) is disposed in a bandage, preferably in bandages for the treatment of the Achilles tendon, the shoulder, the arm, the hand, the trunk, the hip, the knee, the lower leg, the ankle or the foot.

7. Pelotte (1) according to any one of the preceding claims, **characterised in that** the periodic surface structure consists of elevated wave trains (2) that are separated from each other by gaps (3), each of said wave trains (2) forming a recumbent wave parallel to the surface of the pelotte (1).

8. Pelotte (1) according to any one of the preceding claims, **characterised in that** the height profile of at least one wave front exhibits, along its length, a periodic or irregular course, orthogonal to the surface of the pelotte (1), preferably has the shape of superpositions of any number of sine and cosine functions of any phase and wavelength in a Fourier series, more preferably, each with integral multiples of the phase and wavelength of a basis function, more particularly, has the shape of a sine wave, semi-circles, rectangles, zigzag patterns or polygons, with or without rounded corners, in offset arrangement, wherein, preferably, the difference in height along a wave train (2) is from 0.01 mm to 3 cm, preferably from 1 mm to 1 cm.

9. Pelotte (1) according to any one of the preceding claims, **characterised in that** the individual wave trains (2) on the pelotte (1) are uninterrupted along their course.

10. Method of manufacturing a pelotte (1) from a cushion made of an elastic material, **characterised in that** parts of the pelotte's surface or the entire surface of the pelotte (1) exhibits a periodic surface structure according to any one of the preceding claims, and that the wave-like surface structures are incorporated into the surface or applied to the surface, with the periodic surface structures preferably already being contained in the mould employed for producing the pelotte (1), or, after the manufacture of the pelotte (1), being bonded, or welded, to the pelotte (1) in the form of individual wave trains (2) or as a sheet provided with a structure of wave trains (2) or as a textile fabric provided with a structure of wave trains (2).

## Revendications

1. Pelote (1) consistant en un coussin formé d'une matière élastique, des parties de la surface de la pelote (1) ou sa surface toute entière présentant une structure superficielle périodique constituée de série d'ondes en relief (2) qui sont séparées par des intervalles (3) et s'étendent dans un plan X/Y, la direction X et la direction Y étant l'une comme l'autre parallèles à la surface de la pelote (1), la section transversale de la structure superficielle périodique répondant à la forme de demi-cercles intercalés, de rectangles, de profils en dents de scie, d'ondes sinusoïdales ou de polygones à angles arrondis ou non, **caractérisée en ce que** la pelote (1) est pourvue, en plus, d'une perforation.

2. Pelote (1) selon la revendication 1, **caractérisée en ce que** les séries d'ondes (2) ont la forme de fonctions sinus ou de superpositions d'un nombre quelconque de fonctions sinus et cosinus de phase et de longueur d'onde quelconques en série de Fourier, de préférence avec à chaque fois des multiples entiers de la phase et de la longueur d'onde d'une fonction de base, la longueur d'onde d'une série d'ondes (2) étant de préférence comprise entre 1 mm et 30 cm, plus préférablement entre 0,5 cm et 3 cm, et l'amplitude de la série d'ondes (2) étant de préférence comprise entre 0,1 mm et 3 cm, plus préférablement entre 1 mm et 1 cm.

3. Pelote (1) selon l'une des revendications précédentes, **caractérisée en ce que** l'écartement des séries d'ondes (2) est compris entre 0,1 mm et 3 cm, de préférence entre 1 mm et 1 cm, et les séries d'ondes (2), à la différence des intervalles (3), s'élèvent sur une distance comprise entre 0,1 mm et 3 cm, plus préférablement entre 1 mm et 1 cm, par rapport à la surface.

4. Pelote (1) selon l'une des revendications précédentes, **caractérisée en ce que** la structure superficielle périodique est intégrée dans la surface ou appliquée sur la surface, la structure superficielle périodique étant de préférence déjà contenue dans le moule de fabrication de la pelote (1) ou bien étant collée sur la pelote (1) ou soudée avec la pelote (1), après la fabrication de cette dernière (1), sous forme de séries d'ondes individuelles (2) ou en tant que feuille dotée de structures en séries d'ondes (2) ou en tant que textile doté de structures en séries d'ondes (2).

5. Pelote (1) selon l'une des revendications précédentes, **caractérisée en ce que** la structure superficielle périodique contient des matières qui sont choisies dans le groupe comprenant des matières souples non compressibles, des matières souples compressibles et des matières non déformables ou des combinaisons des matières précédemment citées, de préférence des matières plastiques ou des matières naturelles, des tissus à mailles, des tissus ou tissus de séparation, des non-tissés, des produits alvéolaires, des voiles ou matières comparables, plus préférablement des silicones et des caoutchoucs, des mousses de silicone et des mousses polymères, du latex synthétique, du latex naturel, du Néoprène, du polyéthylène, du polypropylène ou un copolymère PP.

6. Pelote (1) selon l'une des revendications précédentes, **caractérisée en ce que** la pelote (1) est située dans un bandage, de préférence des bandages destinés au traitement du tendon d'Achille, de l'épaule, du bras, de la main, du tronc, de la hanche, du genou, de la jambe, de la malléole ou du pied.

7. Pelote (1) selon l'une des revendications précédentes, **caractérisée en ce que** la structure superficielle périodique est constituée de série d'ondes en relief (2) qui sont à chaque fois séparées par des intervalles (3), chacune de ces séries d'ondes (2) formant une onde parallèle à la surface de la pelote (1).

8. Pelote (1) selon l'une des revendications précédentes, **caractérisée en ce que** le profil en hauteur d'au moins un front d'onde a un tracé, périodique ou irrégulier, orthogonal à la surface de la pelote (1) le long de sa longueur, possède de préférence la forme de superpositions d'un nombre quelconque de fonctions sinus et cosinus de phase et de longueur d'onde quelconques en série de Fourier, plus préférablement avec, à chaque fois, des multiples entiers de la phase et de la longueur d'onde d'une fonction de base, possède notamment la forme d'une onde sinusoïdal, de demi-cercles intercalés, de rectangles, de profils en dents de scie ou de polygones à angles arrondis ou non, la différence de hauteur le long d'une série d'ondes (2) étant de préférence comprise entre 0,01 mm et 3 cm, de préférence entre 1 mm et 1 cm.

9. Pelote (1) selon l'une des revendications précédentes, **caractérisée en ce que** les séries d'ondes individuelles (2) sur la pelote (1) ne sont pas interrompues le long de leur trajet.

10. Procédé de fabrication d'une pelote (1) consistant en un coussin formé d'une matière élastique, **caractérisé en ce que** des parties de la surface de la pelote (1) ou sa surface toute entière présentent une structure superficielle périodique selon l'une des revendications précédentes et **en ce que** les structures superficielles ondulées sont intégrées dans la surface ou sont appliquées sur la surface, les structures superficielles périodiques étant de préférence déjà contenues dans le moule de fabrication de la pelote (1) ou bien étant collées sur la pelote (1) ou soudées avec la pelote (1), après la fabrication de cette dernière (1), sous forme de séries d'ondes individuelles (2) ou en tant que feuille dotée de structures en séries d'ondes (2) ou en tant que textile doté de structures en séries d'ondes (2).
